# EUROPÄISCHE PATENTSCHRIFT

(11) **EP 2 236 102 B1**
(45) Veröffentlichungstag und Bekanntmachung des Hinweises auf die Patenterteilung: **06.05.2015**
(21) Anmeldenummer: 09002967.9
(22) Anmeldetag: 03.03.2009
(51) Int. Cl.: A61B 19/00, A61C 19/00

(54) **Reinigungs- oder Pflegegerät zur Reinigung oder Pflege eines medizinischen, insbesondere dentalen Instruments.**
Cleaning or care device for cleaning or caring for a medical, in particular dental instrument
Appareil de nettoyage et d'entretien destiné au nettoyage ou à l'entretien d'un instrument médical, notamment dentaire

(43) Veröffentlichungstag der Anmeldung: 06.10.2010
(73) Patentinhaber: W & H Dentalwerk Bürmoos GmbH, 5111 Bürmoos (AT)
(72) Erfinder: Vedovelli, Renzo, 24060 Brusaporto (IT)
(74) Vertreter: Benda, Ralf

(56) Entgegenhaltungen:
- EP-A- 0 300 945
- JP-A- 2003 047 646
- US-A- 5 227 132
- US-A1- 2004 188 302
- US-B1- 6 594 971

## Beschreibung

Die vorliegende Erfindung betrifft ein Reinigungs- oder Pflegegerät zur Reinigung oder Pflege zumindest eines medizinischen, insbesondere dentalen, Instruments sowie ein Verfahren zur Reinigung oder Pflege eines derartigen Instruments.

Aus der Patentanmeldung WO 2006/126103 A2 ist ein Behälter aus Kunststoff oder Glas bekannt, in den ein zu sterilisierendes oder zu reinigendes Instrument eingefügt werden kann und der an eine Anschlussvorrichtung einer Behandlungskammer eines Sterilisators oder eines Reinigungsgeräts anschließbar ist. Die Anschlussvorrichtung verfügt über Medienleitungen, so dass Reinigungsmedien von der Anschlussvorrichtung in den Behälter und in oder auf das zu reinigende Instrument abgegeben werden können.

Der Vorteil dieses Reinigungssystems ist, dass zur Reinigung eines Instruments nicht die gesamte Behandlungskammer sondern nur das kleinere Volumen des Behälters mit dem Reinigungsmittel gefüllt werden muss. Damit werden eine Verkürzung der Dauer des Reinigungszyklus sowie eine Verringerung der dafür benötigten Ressourcen, wie Energie oder Reinigungsmedien, erzielt.

JP 2003047646 offenbart eine Reinigungskammer mit starren, Wandelementen, die hinzugefügt werden können, um den Innenraum zu reduzieren.

Die Aufgabe der vorliegenden Erfindung besteht darin, ein Reinigungs- oder Pflegegerät zur Reinigung oder Pflege eines medizinischen, insbesondere dentalen, Instruments zu schaffen, das im Vergleich zum Stand der Technik eine noch raschere Reinigung oder Pflege oder eine weitere Verringerung der dafür benötigten Ressourcen ermöglicht.

Gemäß einem Ausführungsbeispiel wird dies durch ein Reinigungs- oder Pflegegerät zur Reinigung oder Pflege zumindest eines medizinischen, insbesondere dentalen, Instruments erreicht, das eine Reinigungskammer zur Aufnahme des zumindest einen zu reinigenden Instruments und eine Medienzufuhr, die ein Reinigungs- oder Pflegemittel in die Reinigungskammer fördert, umfasst, wobei die Reinigungskammer durch eine flexible Außenhülle begrenzt ist, so dass das Raumvolumen der Reinigungskammer veränderbar ist.

Durch das Vorsehen der flexiblen, elastischen Außenhülle und durch die Veränderung des Raumvolumens der Reinigungskammer ist es möglich, die Reinigungskammer an die Außenform oder Gestalt des jeweils zu reinigenden Instruments anzupassen. Die Reinigungskammer weist also keine starre Form und kein gleich bleibendes Raumvolumen auf, wie dies aus dem Stand der Technik bekannt ist, sondern sie ist in ihrem Raumvolumen und insbesondere zusätzlich auch in ihrer Form variabel. Bevorzugt passt sich die Reinigungskammer an die Form des jeweils aufgenommenen Instruments an oder folgt der Form des Instruments. Es sind somit gemäß einem besonders bevorzugten Ausführungsbeispiel keine wesentlichen Leerräume in der Reinigungskammer vorhanden, sondern das Raumvolumen der Reinigungskammer setzt sich nur noch aus dem Volumen des zu reinigenden Instruments und einem geringen Abstand zwischen dem Instrument und der flexiblen Außenhülle zusammen. Auf diese Weise werden die Mengen der zur Reinigung oder Pflege benötigten Ressourcen erheblich reduziert und gleichzeitig die Dauer der Reinigung oder Pflege verkürzt.

Als Reinigungs- oder Pflegegerät im Sinne der Erfindung werden alle Vorrichtungen oder Komponenten von Vorrichtungen bezeichnet, die eine Reinigungsbehandlung, zum Beispiel mit Wasser, mit Druckluft, mit einem Lösungs- oder einem Reinigungsmittel, mit einer Lauge oder mittels Ultraschall, eine Pflegebehandlung, zum Beispiel durch Einbringen eines Schmiermittels, eine Desinfektionsbehandlung oder eine Sterilisationsbehandlung, zum Beispiel mittels Wasserdampf oder mittels Ethylenoxid, oder eine sonstige Behandlung zum Entfernen von Verunreinigungen oder Mikroorganismen an einem medizinischen oder dentalen Instrument ausführen oder ermöglichen. Insbesondere ist das Reinigungs- oder Pflegegerät als Sterilisator, zum Beispiel als Dampfsterilisator, als Thermodesinfektor oder als chemischer Desinfektor ausgebildet.

Das Reinigungs- oder Pflegegerät ist je nach Ausführungsform im Chargenbetrieb, bei dem das Behandlungsmedium eine bestimmte Dauer in der Reinigungskammer verweilt, und / oder im Durchlaufbetrieb, bei dem das Behandlungsmedium kontinuierlich durch die Reinigungskammer fließt ohne darin länger zu verbleiben, betreibbar.

Die zu reinigenden Instrumente können jegliche Art medizinischer, insbesondere dentaler, massiver oder hohler Geräte oder Teile dieser Geräte umfassen, zum Beispiel Spiegel, Pinzetten, Scheren, Zangen, Endoskope, Motore, Kupplungen, Adapter, Hand- und Winkelstücke, Sägeantriebe, Bohrer, Feilen, Sägeblätter, Trokare, Schläuche, Schüsseln, Schalen, Ahlen, Spritzen etc.

Gemäß einem Ausführungsbeispiel sind am Reinigungs- oder Pflegegerät oder damit verbunden Mittel vorgesehen, die die Veränderung des Raumvolumens und / oder der Form der Reinigungskammer bewirken. Diese Mittel sind zum Beispiel als Fördervorrichtung ausgebildet, die ein erstes Arbeitsmedium, zum Beispiel ein Fluid, insbesondere an die Außenseite der flexiblen Außenhülle, fördern. Die Fördervorrichtung umfasst zum Beispiel eine Pumpe für das Arbeitsmedium, einen Kompressor, Leitungen zum Fördern des Arbeitsmediums, Ventile, Drosseln, Schalter oder weitere Stellelemente. Die Fördervorrichtung oder zumindest Teile davon können auch zum Ableiten des Arbeitsmediums, zum Beispiel nach Abschluss der Behandlung des Instruments, verwendet werden.

Als Arbeitsmedium werden bevorzugt ein unter Druck stehendes Gas, zum Beispiel Druckluft, eine Flüssigkeit, zum Beispiel Wasser, oder Schaum verwendet. Das an die Außenseite der flexiblen Außenhülle geförderte erste Arbeitsmedium dehnt aufgrund seines Drucks oder seiner Masse die Außenhülle in Richtung des zu reinigenden Instruments, so dass die Außenhülle sich dem Instrument zumindest nähert oder dieses an einigen Stellen berührt. Wird die Zufuhr des ersten Arbeitsmediums gestoppt und / oder das erste Arbeitsmedium von der Außenseite der flexiblen Außenhülle entfernt, so zieht sich bevorzugt auch die flexible Außenhülle aufgrund ihrer Elastizität vom Instrument zurück oder kehrt besonders bevorzugt wieder in ihre ursprüngliche Ausgangsstellung und zu ihrer ursprünglichen Form zurück.

Die Mittel zur Veränderung des Raumvolumens und / oder der Form der Reinigungskammer können alternativ oder zusätzlich zu der oben genannten Fördervorrichtung auch zumindest ein bewegliches mechanisches Element umfassen, zum Beispiel einen verschiebbaren Kolben oder Stift, welches die flexible Außenhülle, bevorzugt durch direkten Kontakt, in Richtung des Instruments bewegt und / oder davon wegbewegt. Zur Bewegung des zumindest einen beweglichen mechanischen Elements ist dieses bevorzugt mit einem Antrieb verbunden, zum Beispiel mit einem pneumatischen, hydraulischen oder elektromotorischen Antrieb. Besonders bevorzugt ist das zumindest eine bewegliche mechanische Element teleskopartig aufgebaut.

Gemäß einem Ausführungsbeispiel ist die flexible Außenhülle an einer starren, bevorzugt metallischen oder aus Kunststoff bestehenden, Wand befestigt. Die starre Wand ist zum Beispiel als Kammer oder Gehäuse ausgebildet, das die flexible Außenhülle umgibt, insbesondere als Kammer oder Gehäuse einer Reinigungs- oder Pflegeeinheit, zum Beispiel eines Sterilisators. Alternativ ist die starre Wand Teil eines Reinigungs- / Pflege- und Aufbewahrungsbehälters, der bevorzugt in eine Reinigungs- oder Pflegeeinheit, zum Beispiel einen Sterilisator, einbringbar und daraus entnehmbar ist. Der Innenraum des Reinigungs- / Pflege- und Aufbewahrungsbehälters bildet in diesem Fall die Reinigungs-/Pflege- und Aufbewahrungskammer, in die das Instrument zur Reinigung / Pflege eingebracht wird und in der es bis zur neuerlichen Benutzung aufbewahrt wird. Bevorzugt ist die starre Wand dabei als Behälterdeckel des Reinigungs- / Pflege- und Aufbewahrungsbehälters ausgebildet.

Umgibt die starre Wand die flexible Außenhülle, so münden gemäß einem Ausführungsbeispiel die Fördermittel für das erste Arbeitsmedium zwischen der flexiblen Außenhülle und der starren Wand, so dass das erste Arbeitsmedium zwischen die flexible Außenhülle und die starre Wand förderbar ist. Damit ist in vorteilhafter Weise ein Speicherraum zwischen der starren Wand und der flexiblen Außenhülle geschaffen, in den das erste Arbeitsmedium eingebracht, während der Behandlung des Instruments gespeichert und wieder abgeführt werden kann, womit auch die Menge des zu fördernden ersten Arbeitsmediums und die für die Förderung notwendige Energie deutlich reduziert werden. Besonders bevorzugt umfassen die Fördermittel für das erste Arbeitsmedium zusätzlich ein Sperrelement, zum Beispiel ein Steuerventil oder einen Schalthahn, das den Speicherraum nach dem Befüllen mit dem ersten Arbeitsmedium von der Umgebung abschließt, so dass der Speicherraum nicht kontinuierlich, sondern diskontinuierlich, bevorzugt nur einmal vor Beginn der Behandlung des Instruments, mit dem ersten Arbeitsmedium gefüllt wird.

Gemäß einem Ausführungsbeispiel ist die flexible Außenhülle durch eine elastische Folie oder durch eine elastische Membran, insbesondere aus Kunststoff, Kautschuk oder Gummi, gebildet, die insbesondere für Wasser und / oder Wasserdampf und / oder Druckluft und / oder Mikroorgansimen undurchlässig ist.

Gemäß einem bevorzugten Ausführungsbeispiel umfasst das Reinigungs- oder Pflegegerät eine Abstandhaltervorrichtung, die das Anliegen der flexiblen Außenhülle am Instrument oder den Kontakt zwischen dem Instrument und der flexiblen Außenhülle verhindert oder dem zu festen Anliegen der flexiblen Außenhülle am Instrument entgegenwirkt, so dass das Behandlungsmedium die Außenseite oder Oberfläche des Instruments umfließen kann. Die Abstandhaltervorrichtung weist zum Beispiel Abstandhalter auf, die an der Innenseite der flexiblen Außenhülle, die gleichzeitig die Innenseite der Reinigungskammer bildet, angeordnet sind und die dazu ausgebildet sind, zumindest teilweise an dem zu reinigenden Instrument anzuliegen. Die Abstandhalter können insbesondere durch Noppen oder Leisten gebildet sein. Die Abstandhalter sind in regelmäßigen oder unregelmäßigen Abständen angeordnet und bewirken, dass die flexible Außenhülle das zu reinigende Instrument nicht oder nicht vollständig berührt.

Damit das Behandlungsmedium auch an jene Stellen der Außenseite oder Oberfläche des Instruments gelangt, die von den Abstandhaltern kontaktiert werden, sind in einem besonders bevorzugten Ausführungsbeispiel Mittel zum Bewegen des in der Reinigungskammer aufgenommenen Instruments relativ zur flexiblen Außenhülle vorgesehen. Selbstverständlich können durch diese Bewegungsmittel auch Oberflächenabschnitte des Instruments von der flexiblen Außenhülle getrennt werden, falls die Außenhülse Instrumentenabschnitte berührt. Die Bewegungsmittel umfassen zum Beispiel ein Anschlussstück, an dem das zu reinigende Instrument befestigbar ist, oder eine Aufnahme, in die das zu reinigende Instrument einsetzbar ist, und die in Rotation oder Vibration versetzbar, verschwenkbar oder verschiebbar sind. Das Anschlussstück ist bevorzugt als rohr- oder zapfenförmiges Kupplungselement ausgebildet. Das Anschlussstück oder die Aufnahme sind, gegebenenfalls über Verbindungselemente wie Wellen, Getriebe oder Schwingungsüberträger, mit einem Antrieb zum Erzeugen der Bewegung verbunden, zum Beispiel einem Elektromotor oder einem Schwingungsgenerator.

Alternativ oder zusätzlich umfasst die Abstandhaltervorrichtung eine Fördervorrichtung für ein zweites Arbeitsmedium, insbesondere ein Fluid, die in die Reinigungskammer mündet und die das zweite Arbeitsmedium in die Reinigungskammer, zwischen das zu reinigende Instrument und die flexiblen Außenhülle, einbringt. Das zweite Arbeitsmedium umgibt oder umströmt das Instrument oder durchströmt die Reinigungskammer, bevorzugt jeweils kontinuierlich, und verhindert somit zumindest zu gewissen Zeitpunkten und / oder an gewissen Stellen des zu reinigenden Instruments das Anliegen oder das zu feste Anliegen der flexiblen Außenhülle am Instrument. Die Fördervorrichtung ist entweder als separate, ausschließlich das zweite Arbeitsmedium transportierende Fördervorrichtung ausgebildet oder sie ist Teil einer gemeinsamen Fördervorrichtung zur Förderung des ersten und des zweiten Arbeitsmediums oder zur Förderung des zweiten Arbeitsmediums und zumindest eines Reinigungs- oder Pflegemittels. Im Falle einer gemeinsamen Fördervorrichtung werden zumindest Teile der Fördervorrichtung, zum Beispiel Leitungen, Stellelemente, Schaltelemente, Pumpen etc., gemeinsam für das zweite Arbeitsmedium und das andere Medium verwendet.

Besonders bevorzugt umfasst die Abstandhaltervorrichtung neben der Fördervorrichtung für das zweite Arbeitsmedium zusätzlich eine Steuer- und / oder Regelvorrichtung, die den in der Speicher- und / oder Reinigungskammer herrschenden Druck derart steuert oder regelt, dass die flexible Außenhülle das zu reinigende Instrument nicht oder nur teilweise berührt. Dazu umfasst die Steuer- und / oder Regelvorrichtung einen oder mehrere Drucksensoren, wobei zumindest jeweils ein Drucksensor in der Reinigungskammer und in dem Speicherraum angeordnet ist. Die Drucksensoren detektieren die Drücke in der Reinigungskammer und in dem Speicherraum und leiten entsprechende Drucksensorsignale an die Steuer- und / oder Regelvorrichtung. Die Steuer- und / oder Regelvorrichtung vergleicht die Drucksensorsignale miteinander und / oder mit vorbestimmten Druckvergleichswerten und steuert und / oder regelt zumindest einen Betriebsparameter eines der beiden oder beider Arbeitsmedien, insbesondere den Volumenstrom und / oder den Druck, derart, dass die flexible Außenhülle das zu reinigende Instrument zumindest an einer Stelle nicht berührt. Alternativ weist die Steuer- und / oder Regelvorrichtung andere Sensoren auf, zum Beispiel einen oder mehrere Berührungssensoren, die einen Kontakt zwischen dem Instrument und der flexiblen Außenhülle detektieren, oder Dehnungssensoren, die jeweils an der flexiblen Außenhülle angeordnet sind und deren Sensorsignale wie oben beschrieben für die Steuerung / Regelung eines Betriebsparameters zumindest eines der beiden Arbeitsmedien dienen.

Die Steuer- und / oder Regelvorrichtung zur Förderung des ersten und / oder des zweiten Arbeitsmediums und gegebenenfalls auch zur Abfuhr des ersten und / oder des zweiten Arbeitsmediums umfasst zum Beispiel zumindest ein Ventil und / oder zumindest einen Sensor, insbesondere einen Drucksensor oder einen Durchflusssensor, und / oder zumindest einen Mikrokontroller. Die Steuer- und / oder Regelvorrichtung überwacht, steuert und / oder regelt bevorzugt auch den gesamten Ablauf des Reinigungs- oder Pflegeprozesses.

Ein Verfahren zur Reinigung oder Pflege eines medizinischen, insbesondere dentalen, Instruments mit einem im Vorhergehenden beschriebenen Reinigungs- oder Pflegegerät umfasst den Schritt, dass nach dem Einbringen des Instruments in die Reinigungskammer das Volumen der Reinigungskammer verändert wird. Dazu wird ein erstes Arbeitsmedium, insbesondere ein Fluid, in Richtung oder an die Außenseite der flexiblen Außenhülle gefördert. Bevorzugt wird das Volumen der Reinigungskammer derart verändert, dass sich die Form der Reinigungskammer im Wesentlichen der Form des Instruments angleicht, ihr folgt oder entspricht oder die flexible Außenhülle zumindest teilweise am Instrument anliegt.

Die Erfindung wird nachfolgend anhand bevorzugter Ausführungsbeispiele und Bezug nehmend auf die beigefügten Zeichnungen erläutert:
Die Figuren 1A und 1B zeigen in schematischen Darstellungen ein erstes Ausführungsbeispiel einer Reinigungskammer eines Reinigungs- oder Pflegegeräts in leerem Zustand (Figur 1A) und beladenem Zustand während der Reinigung oder Pflege (Figur 1 B).
Die Figuren 2A und 2B zeigen in schematischen Darstellungen ein zweites Ausführungsbeispiel einer Reinigungskammer eines Reinigungs- oder Pflegegeräts in leerem Zustand (Figur 2A) und beladenem Zustand während der Reinigung oder Pflege (Figur 2B).
Figur 3 zeigt einen vergrößerten Ausschnitt der Figur 2B mit einem Teil des zu reinigenden Instruments und mit der in ihrem Raumvolumen und in ihrer Form an das Instrument angepassten Reinigungskammer.
Die Figuren 4A und 4B zeigen in schematischen Darstellungen ein drittes Ausführungsbeispiel einer Reinigungskammer eines Reinigungs- oder Pflegegeräts in beladenem Zustand, mit einem ersten Raumvolumen der Reinigungskammer (Figur 4A) und einem zweiten, verringerten Raumvolumen während der Reinigung oder Pflege des Instruments (Figur 4B).

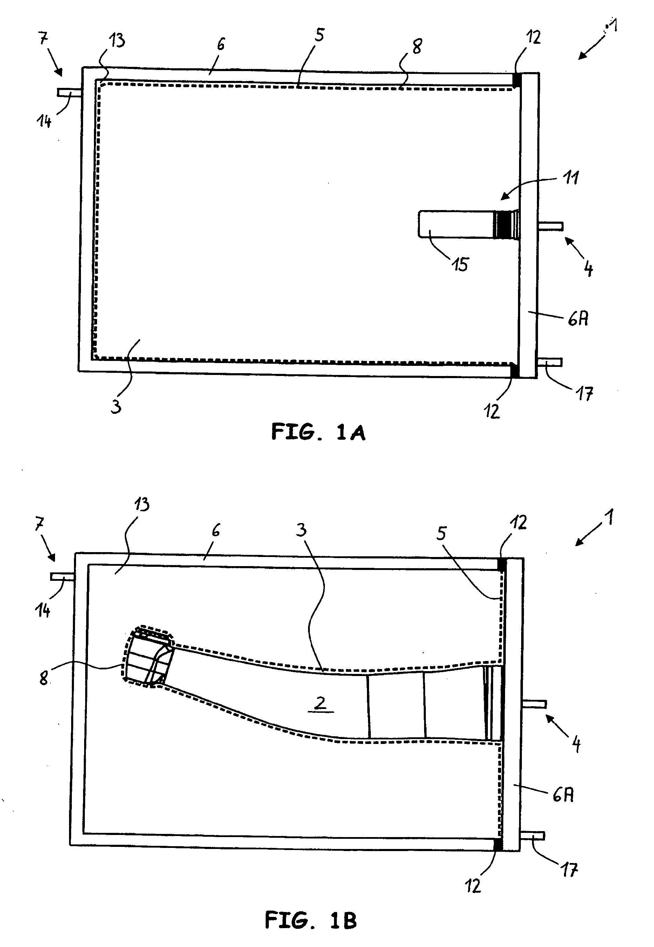

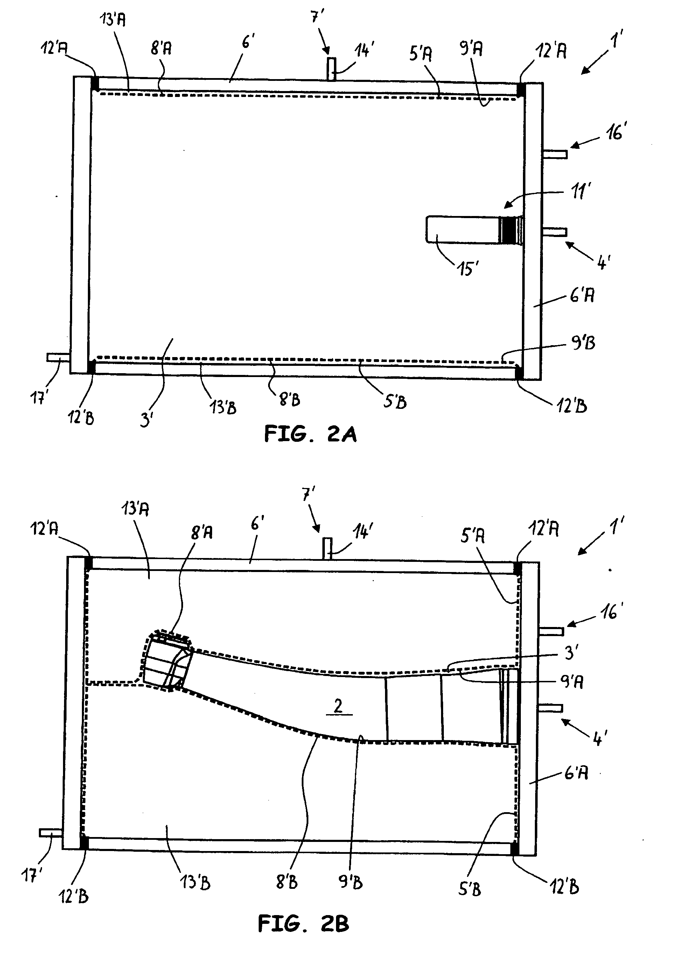

Die in den Figuren 1A, 1B, 2A und 2B dargestellten Reinigungs- oder Pflegegeräte 1, 1' weisen mehrere starre, zum Beispiel metallische, Wände 6, 6' auf, die zusammen ein Außengehäuse der Reinigungs- oder Pflegegeräte 1, 1' bilden. An einer Seitenwand ist ein vom restlichen Gehäuse entfernbarer Deckel 6A, 6'A oder eine verschwenkbare Tür vorgesehen, so dass in das Innere des Gehäuses ein oder mehrere zu reinigende Instrumente 2 eingebracht und daraus entnommen werden können.

Im Inneren des Gehäuses ist eine flexible Außenhülle 5, 5'A, 5'B vorgesehen, die an zwei oder mehr Punkten 12, 12'A, 12'B oder Verbindungsabschnitten an festen Bauteilen des Reinigungs- oder Pflegegeräts 1, 1' befestigt ist, insbesondere an der Innenseite des Außengehäuses. Der überwiegende Teil der flexiblen Außenhülle 5, 5'A, 5'B ist jedoch lose im Gehäuse angeordnet und frei darin beweglich. Die flexible Außenhülle 5, 5'A, 5'B besteht entweder aus einer einzigen sackartigen oder zylindrischen, elastischen Folie oder Membran (siehe Figuren 1A, 1B) oder sie umfasst zwei oder mehr von einander getrennte Abschnitte 5'A, 5'B, die jeweils unabhängig voneinander an unterschiedlichen Punkten 12'A, 12'B in den Reinigungs- oder Pflegegeräten 1, 1' befestigt sind (siehe Figuren 2A, 2B).

Der von der flexiblen Außenhülle 5, 5'A, 5'B umschlossene Raum bildet eine Reinigungskammer 3, 3', in die ein oder mehrere zu reinigende Instrumente 2 einbringbar sind. Aufgrund der Flexibilität und Elastizität der Außenhülle 5, 5'A, 5'B sind zumindest das Raumvolumen und bevorzugt auch die Form der Reinigungskammer 3, 3' veränderbar.

Die Raumvolumen- oder Formänderung der Reinigungskammer 3, 3' wird durch ein erstes Arbeitsmedium bewirkt, insbesondere durch ein Fluid, bevorzugt durch eine Flüssigkeit, das über Fördermittel oder eine Fördervorrichtung 7, 7' zur Förderung des ersten Arbeitsmediums in einen Zwischen- oder Speicherraum 13, 13'A, 13'B förderbar ist. Zumindest ein Teil der Fördervorrichtung 7, 7' mündet in den Speicherraum 13, 13'A, 13'B. Die Fördervorrichtung 7, 7' umfasst zum Beispiel eine Leitung 14, 14', die das Gehäuse der Reinigungs- oder Pflegegeräte 1, 1' durchsetzt, einen Kompressor, Druckregel- oder Steuerventile. Das in den Speicherraum 13, 13'A, 13'B und damit an die Außenseite 8, 8'A, 8'B der flexiblen Außenhülle 5, 5'A, 5'B geförderte erste Arbeitsmedium bewirkt durch seinen Druck eine Formveränderung oder Dehnung der flexiblen Außenhülle 5, 5'A, 5'B.

Die Figuren 1A und 2A zeigen die flexible Außenhülle 5, 5'A, 5'B in ihrer Ausgangsposition vor der Förderung des ersten Arbeitsmediums. Die flexible Außenhülle 5, 5'A, 5'B erstreckt sich entlang der Wände 6, 6', so dass die Reinigungskammer 3, 3' im Vergleich zum Speicherraum 13, 13'A, 13'B ein deutlich größeres Raumvolumen aufweist, bevorzugt ihr maximales Raumvolumen einnimmt, wodurch ein komfortables Einbringen des zu reinigenden Instruments 2 für den Anwender gewährleistet ist.

Die Situation nach dem Einbringen des Instruments 2 in das Reinigungs- oder Pflegegerät 1, 1' und nach der Förderung des ersten Arbeitsmediums in den Speicherraum 13, 13'A, 13'B und gegebenenfalls dessen Verschluss durch ein Sperrelement, zum Beispiel ein Ventil, ist in den Figuren 1B, 2B dargestellt. Die flexible Außenhülle 5, 5'A, 5'B folgt der Form des Instruments 2, wodurch sich das Raumvolumen der Reinigungskammer 3, 3' verringert und das Raumvolumen des Speicherraum 13, 13'A, 13'B vergrößert hat. Insbesondere ist jener Volumenanteil der Reinigungskammer 3, 3', der nicht vom Instrument 2 ausgefüllt ist und in dem ein Reinigungs- oder Pflegemittel, zum Beispiel Wasserdampf oder ein Desinfektionsmittel, die Oberfläche des Instruments 2 umgibt, deutlich verkleinert, so dass die Menge des für die Reinigung oder Pflege benötigten Behandlungsmittels und die Dauer des Füllens des Behandlungsmittels in die Reinigungskammer 3, 3' oder des Entfernens des Behandlungsmittels aus der Reinigungskammer 3, 3' auf ein Minimum reduzierbar sind. Dies ist insbesondere bei Sterilisatoren, bei denen die gesamte Reinigungskammer 3, 3' während eines Sterilisationszykluses mehrmals mit Wasserdampf zu befüllen und anschließend davon wieder zu evakuieren ist, von erheblichem Vorteil.

Nach Abschluss der Reinigung oder Pflege des Instruments 2 wird das erste Arbeitsmedium über die Fördermittel 7 oder über andere Fördermittel und Leitungen aus der Reinigungskammer 3, 3' abgeleitet, wodurch die flexible Außenhülle 5, 5'A, 5'B im Wesentlichen wieder in ihre in den Figuren 1A, 2A dargestellte Ausgangsposition und -form zurückkehrt. Der Speicherraum 13, 13'A, 13'B und die Reinigungskammer 3, 3' weisen somit ein Gesamtvolumen V auf, das sich aus den während eines Behandlungs- oder Pflegezykluses variablen Volumina V1 des Speicherraums 13, 13'A, 13'B und V2 der Reinigungskammer 3, 3' zusammensetzt.

Die Befestigung des Instruments 2 in dem Reinigungs- oder Pflegegerät 1, 1' erfolgt gemäß den Ausführungsbeispielen der Figuren 1A - 2B durch ein mit der Wand 6, 6' verbundenes, zapfenförmiges Steckkupplungselement 15, 15', das in eine entsprechende rohrförmige Aufnahme des Instruments 2 einfügbar ist. Das Steckkupplungselement 15, 15' ist bevorzugt als hohler Schaft ausgebildet oder umfasst eine oder mehrere Leitungen, die über die Medienzufuhr 4, 4', die zum Beispiel mehrere Medienleitungen umfasst, mit Medienquellen für die Reinigungs- oder Pflegemittel verbunden sind. Über das Steckkupplungselement 15, 15' wird das Reinigungs- oder Pflegemittel in die Reinigungskammer 3, 3' und falls das Instrument 2 Hohlräume, Leitungen, Kanäle etc. in seinem Inneren aufweist, die ebenfalls gereinigt oder gepflegt werden sollen, auch direkt in das Innere des Instruments 2 geleitet. Anstelle des Steckkupplungselements 15, 15' können selbstverständlich andere Befestigungsvorrichtungen für zu reinigende Instrumente vorgesehen sein, zum Beispiel Körbe oder Aufnahmen zum Einlegen des Instruments 2.

In den Figuren 2A, 2B umfasst das Reinigungs- oder Pflegegerät 1' zusätzlich oder alternativ zu dem der Reinigungs- oder Pflegemittelzufuhr dienenden Steckkupplungselement 15' eine zweite Medienzufuhr 16' mit einer Medienleitung, die direkt in die Reinigungskammer 3' mündet. Über diese ebenfalls mit einer Medienquelle verbundene zweite Medienzufuhr 16' kann ein Reinigungs- oder Pflegemittel, das insbesondere der Außenreinigung oder -pflege des Instruments 2 dient, in die Reinigungskammer 3' geleitet werden. Alternativ oder zusätzlich kann über die zweite Medienzufuhr 16' ein zweites Arbeitsmedium, insbesondere ein Fluid, in die Reinigungskammer 3' eingebracht werden, das zumindest teilweise das Anliegen der flexiblen Außenhülle 5'A, 5'B am Instrument 2 oder den Kontakt zwischen dem Instrument 2 und der flexiblen Außenhülle 5'A, 5'B verhindert oder dem zu festen Anliegen der flexiblen Außenhülle 5'A, 5'B am Instrument 2 entgegenwirkt.

Selbstverständlich ist es auch möglich der, Reinigungskammer 3, 3' das zweite Arbeitsmedium durch das Steckkupplungselement 15, 15' oder durch entsprechende Befestigungsvorrichtungen für das Instrument 2 zuzuleiten. Das Steckkupplungselement 15, 15' weist in diesem Fall entweder eine direkte Verbindung für das zweite Arbeitsmedium in die Reinigungskammer 3, 3' auf oder das zweite Arbeitsmedium strömt durch das Instrument 2 und durch am Instrument 2 vorgesehene Öffnungen, zum Beispiel der Werkzeugaufnahmeöffnung oder Sprayöffnungen, in die Reinigungskammer 3, 3'.

Über eine Medienabfuhr 17, 17' verlassen die Reinigungs- oder Pflegemittel die Reinigungskammer 3, 3'. Die Medienabfuhr 17, 17' umfasst zum Beispiel eine Saugpumpe, Steuer- oder Rückschlagventile, Leitungen oder Behälter zum Sammeln der verbrauchten Behandlungsmedien.

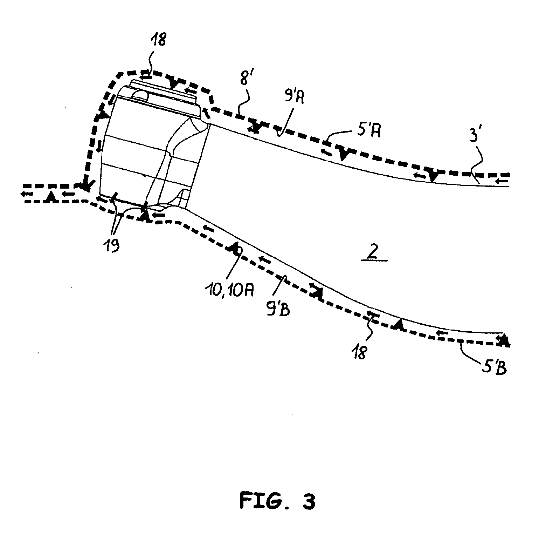

Anhand des in der Figur 3 dargestellten vergrößerten Ausschnitts der Figur 2B ist zu erkennen, dass an der Innenseite 9'A, 9'B der flexible Außenhülle 5'A, 5'B Abstandhalter 10 in Form von dreieckigen Noppen 10A vorgesehen sind. Bevorzugt sind die Abstandhalter 10 integraler Teil der flexiblen Außenhülle 5'A, 5'B. Sie verhindern ein Anliegen der Folie oder Membran der flexiblen Außenhülle 5'A, 5'B am Instrument 2 und ermöglichen somit den durch die Pfeile 18 angedeuteten Fluss des Reinigungs- oder Pflegemittel durch die Reinigungskammer 3' entlang der Außenseite des Instruments 2. Die Pfeile 19 weisen auf den Austritt von Reinigungs- oder Pflegemittel aus dem Inneren des Instruments 2 hin.

Damit auch jene Abschnitte der Oberfläche des Instruments 2, die von den Abstandhaltern 10 bedeckt oder kontaktiert sind oder an denen gegebenenfalls die flexible Außenhülle 5'A, 5'B anliegt, mit Reinigungs- oder Pflegemittel in Berührung kommen, sind des Weiteren Mittel 11, 11' zum Bewegen des in der Reinigungskammer 3, 3' aufgenommenen Instruments 2 relativ zur flexiblen Außenhülle 5, 5'A, 5'B vorgesehen. Die Bewegungsmittel 11, 11' umfassen neben dem oben beschriebenen, in Rotation, eine Längsbewegung oder in Vibration versetzbaren Steckkupplungselement 15, 15' oder anderen Befestigungsvorrichtungen für das Instrument 2 zum Beispiel Wellen, Getriebe, Schwingungsüberträger oder einen Antrieb zum Erzeugen der Bewegung, zum Beispiel einen Elektromotor oder einen Schwingungsgenerator.

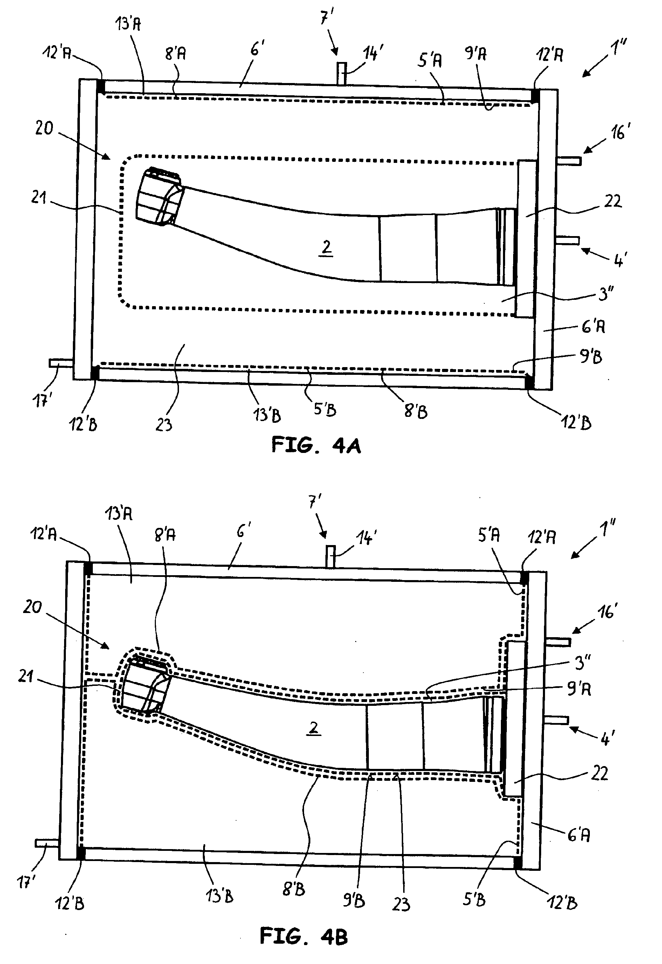

Das Reinigungs- oder Pflegegerät 1" gemäß den Figuren 4A und 4B umfasst zumindest einen Behälter 20, bevorzugt zusätzlich auch ein Reinigungs- oder Pflegegerät, das ist im Wesentlichen gleich aufgebaut wie das Reinigungs- oder Pflegegerät 1' der Figuren 2A, 2B, so dass gleiche Bauteile mit denselben Bezugszeichen bezeichnet sind. Im Unterschied zu den im Vorherstehenden beschriebenen Reinigungs- oder Pflegegeräten ist die Reinigungskammer 3" des Reinigungs- oder Pflegegeräts 1" durch den Innenraum des in das Reinigungs- oder Pflegegerät 1" einbringbaren und daraus entnehmbaren Behälters 20 gebildet. Der Behälter 20 umfasst eine, zum Beispiel zylindrisch geformte, Außenwand, die zumindest teilweise durch eine flexible Außenhülle 21 und einen mit der Außenhülle 21 lösbar verbindbaren Deckel 22 gebildet ist.

An der der Reinigungskammer 3" zugewandten Innenseite des Deckels 22 ist eine Befestigungsvorrichtung für das zu reinigende Instrument 2 vorgesehen, zum Beispiel ein aus den Figuren 1A und 2A bekanntes Steckkupplungselement 15, 15'. An der von der Reinigungskammer 3" abgewandten Außenseite des Deckels 22 ist zumindest ein Medienanschluss vorgesehen, der direkt oder indirekt über die Medienzufuhr 4', 16' des Deckels 6'A mit einer oder mehreren Medienquellen verbindbar oder verbunden ist und der des Weiteren mit der Befestigungsvorrichtung für das zu reinigende Instrument 2 verbunden ist, so dass zumindest ein Reinigungs- oder Pflegemittel und / oder ein zweites Arbeitsmedium in das Instrument 2 und / oder in die Reinigungskammer 3" förderbar ist. Die Ableitung der in die Reinigungskammer 3" geförderten Medien erfolgt ebenfalls über den Deckel 22 durchsetzende Medienleitungen.

Der Behälter 20 umfasst somit eine Reinigungskammer 3" zur Aufnahme des zumindest einen zu reinigenden Instruments 2 und eine Medienzufuhr, durch die ein Reinigungs- oder Pflegemittel in die Reinigungskammer 3" förderbar ist, wobei die Reinigungskammer 3" durch eine flexible Außenhülle 21 begrenzt ist, so dass das Raumvolumen der Reinigungskammer 3" veränderbar ist. Wie schon weiter oben für die Reinigungs- oder Pflegegeräte 1, 1' dargestellt, ist aufgrund der Flexibilität der Außenhülle 21 des Behälters 20 das Volumen der Reinigungskammer 3" variabel. Die Veränderung des Raumvolumens erfolgt in gleicher Weise wie für das Reinigungs- oder Pflegegerät 1' beschrieben, also durch Einbringen eines ersten Arbeitsmediums mittels der Fördervorrichtung 7' in den Speicherraum 13'A, 13'B, wodurch sich die erste flexible Außenhülle 5'A, 5'B verformt und das Volumen des Speicherraums zunimmt, bis die flexible Außenhülle 5'A, 5'B die flexible Außenhülle 21 des Behälters 20 kontaktiert und diese verdrängt oder derart verformt, dass das Raumvolumen der Reinigungskammer 3" abnimmt (siehe Figur 4B). Durch Ablassen des ersten Arbeitsmediums aus dem Speicherraum 13'A, 13'B kehren beide flexiblen Außenhüllen 21, bevorzugt selbsttätig, im Wesentlichen in ihre Ausgangspositionen zurück.

Der Behälter 20 weist gemäß einem Ausführungsbeispiel eine Abstandhaltervorrichtung zur zumindest teilweisen Beabstandung der flexiblen Außenhülle 21 von dem Instrument 2 auf, wobei die Abstandhaltervorrichtung zum Beispiel an der Innenseite der flexiblen Außenhülle 21 vorgesehene Abstandhalter, insbesondere Noppen oder Leisten, oder eine in die Reinigungskammer 3" mündende Fördervorrichtung 16' für ein zweites Arbeitsmedium umfasst. Der Aufbau dieser Abstandhaltervorrichtung entspricht dem Aufbau der im Vorherstehenden beschriebenen Abstandhaltervorrichtungen.

Insbesondere wenn als erstes Arbeitsmedium ein gasförmiges Medium verwendet wird, zum Beispiel Druckluft, kann gemäß einem Ausführungsbeispiel die erste flexible Außenhülle 5'A, 5'B entfallen, so dass das erste Arbeitsmedium über die Fördervorrichtung 7' direkt zur Außenseite der flexiblen Außenhülle 21 des Behälters 20 gefördert wird, bevorzugt durch Abgabe des ersten Arbeitsmediums in den Innenraum 23 des durch die Wände 6' gebildeten Außengehäuses.

Der Vorteil der Verwendung des Behälters 20 besteht insbesondere darin, dass der Behälter 20 nicht nur zur Aufbewahrung des Instruments 2 während der Reinigung oder Pflege dient, sondern, dass das Instrument 2 nach Abschluss der Reinigung oder Pflege bis zu seiner erneuten Benutzung in dem Behälter 20 verbleiben kann. Der Behälter 20 ist somit aufgrund seiner Undurchlässigkeit für Wasser und / oder Wasserdampf und / oder Druckluft und / oder Mikroorganismen auch ein Schutz- und Aufbewahrungsbehälter für das gereinigte oder gepflegte Instrument 2.

Zur Reinigung oder Pflege wird der Deckel 22 vom Behälter 20 gelöst, das Instrument 2 an der am Deckel 22 vorgesehene Befestigungsvorrichtung, zum Beispiel an dem Steckkupplungselement 15, 15', befestigt und der Deckel 22 mit dem Instrument 2 wieder auf dem Behälter 20 befestigt, so dass das Instrument 2 in dem die Reinigungskammer 3" bildenden Innenraum des Behälters 20 aufgenommen ist. Anschließend wird der Behälter 20 mit dem Instrument 2 in den Innenraum 23 des Reinigungs- und Pflegegeräts 1' eingebracht und mit der oder den Medienquellen verbunden. Nach Abschluss der Reinigung oder Pflege, bei der das Volumen der Reinigungskammer 3" verändert wird, wird die Verbindung des Behälters 20 mit den Medienquellen getrennt, der Behälter 20 mit dem Instrument 2 aus dem Innenraum 23 entnommen und bis zur Verwendung des Instruments 2 verschlossen verwahrt.

## Patentansprüche

1. Reinigungs- oder Pflegegerät (1, 1', 1") zur Reinigung oder Pflege zumindest eines medizinischen, insbesondere dentalen, Instruments (2) mit einer Reinigungskammer (3, 3', 3") zur Aufnahme des zumindest einen zu reinigenden Instruments (2) und mit einer Medienzufuhr (4, 4', 16'), durch die ein Reinigungs- oder Pflegemittel in die Reinigungskammer (3, 3', 3") förderbar ist, **dadurch gekennzeichnet, dass** die Reinigungskammer (3, 3', 3") durch eine flexible Außenhülle (5, 5'A, 5'B, 21) begrenzt ist, so dass das Raumvolumen der Reinigungskammer (3, 3', 3") veränderbar ist, wobei die flexible Außenhülle (5, 5'A, 5'B, 21) an einer starren, bevorzugt metallischen oder aus Kunststoff bestehenden, Wand (6, 6') befestigt ist.

2. Reinigungs- oder Pflegegerät (1, 1', 1") nach Anspruch 1, **dadurch gekennzeichnet, dass**
die starre Wand (6, 6') als Kammer oder Gehäuse ausgebildet ist, die / das die flexible Außenhülle (5, 5'A, 5'B, 21) umgibt, insbesondere als Kammer oder Gehäuse einer Reinigungs- oder Pflegeeinheit

3. Reinigungs- oder Pflegegerät (1, 1', 1") nach Anspruch 1 oder 2, **dadurch gekennzeichnet, dass**
Mittel zur Veränderung des Raumvolumens der Reinigungskammer (3, 3', 3") vorgesehen sind, wobei diese Mittel zum Beispiel eine Fördervorrichtung (7, 7') zur Förderung eines Arbeitsmediums, insbesondere an die Außenseite (8, 8'A, 8'B) der flexiblen Außenhülle (5, 5'A, 5'B, 21), und / oder zumindest ein bewegliches mechanisches Element umfassen.

4. Reinigungs- oder Pflegegerät (1, 1', 1") nach Anspruch 3, **dadurch gekennzeichnet, dass** die Mittel zur Veränderung des Raumvolumens der Reinigungskammer (3, 3' 3") eine Fördervorrichtung (7, 7') zur Förderung eines Arbeitsmediums zwischen die starre Wand (6, 6') und die flexible Außenhülle (5, 5'A, 5'B, 21) umfassen, so dass das Arbeitsmedium zwischen die starre Wand (6, 6') und die flexible Außenhülle (5, 5'A, 5'B, 21) förderbar ist.

5. Reinigungs- oder Pflegegerät (1, 1', 1 ") nach einem der vorhergehenden Ansprüche, **dadurch gekennzeichnet, dass**
die flexible Außenhülle (5, 5'A, 5'B, 21) durch eine elastische Folie oder durch eine elastische Membran, insbesondere aus Kunststoff oder Gummi, gebildet ist.

6. Reinigungs- oder Pflegegerät (1, 1', 1") nach Anspruch 5, **dadurch gekennzeichnet, dass**
die Folie oder Membran undurchlässig für Wasser und / oder Wasserdampf und / oder Druckluft und / oder Mikroorganismen ist.

7. Reinigungs- oder Pflegegerät (1, 1', 1 ") nach einem der vorhergehenden Ansprüche, **dadurch gekennzeichnet, dass**
eine Abstandhaltervorrichtung zur zumindest teilweisen Beabstandung der flexiblen Außenhülle (5, 5'A, 5'B, 21) von dem Instrument (2) vorgesehen ist, wobei die Abstandhaltervorrichtung zum Beispiel an der Innenseite (9'A, 9'B) der flexiblen Außenhülle (5, 5'A, 5'B, 21) vorgesehene Abstandhalter (10), insbesondere Noppen (10A) oder Leisten, oder eine in die Reinigungskammer (3, 3') mündende Fördervorrichtung (16') für ein zweites Arbeitsmedium umfasst.

8. Reinigungs- oder Pflegegerät (1, 1', 1 ") nach einem der vorhergehenden Ansprüche, **dadurch gekennzeichnet, dass**
Mittel (11, 11') zum Bewegen des in der Reinigungskammer (3, 3', 3") aufgenommenen Instruments (2) relativ zur flexiblen Außenhülle (5, 5'A, 5'B, 21) vorgesehen sind, um Oberflächenabschnitte des Instruments (2) von der flexiblen Außenhülle (5, 5'A, 5'B, 21) oder von einem Abstandhalter (10) zu trennen.

9. Reinigungs- oder Pflegegerät (1, 1', 1 ") nach einem der vorhergehenden Ansprüche, **gekennzeichnet durch**
eine Steuer- und / oder Regelvorrichtung zur Steuerung und / oder Regelung der Förderung zumindest eines Arbeitsmediums oder zum Abführen zumindest eines Arbeitsmediums.

10. Reinigungs- oder Pflegegerät (1, 1', 1 ") nach Anspruch 9, **dadurch gekennzeichnet, dass**
die Steuer- und / oder Regelvorrichtung zumindest ein Ventil und / oder zumindest einen Sensor, insbesondere einen Drucksensor oder einen Durchflusssensor, und / oder zumindest einen Mikrokontroller umfasst.

11. Reinigungs- oder Pflegegerät (1, 1', 1 ") nach einem der vorhergehenden Ansprüche, **dadurch gekennzeichnet, dass**
das Reinigungs- oder Pflegegerät (1, 1', 1") als Sterilisator, insbesondere als Dampfsterilisator, als Thermodesinfektor oder als chemischer Desinfektor ausgebildet ist.

12. Reinigungs- oder Pflegegerät (1, 1', 1 ") nach einem der vorhergehenden Ansprüche, **dadurch gekennzeichnet, dass**
die Reinigungskammer (3, 3', 3") durch den Innenraum eines in das Reinigungs- oder Pflegegerät (1, 1', 1") einbringbaren und daraus entnehmbaren Behälters (20) gebildet ist.

13. Verfahren zur Reinigung oder Pflege eines medizinischen, insbesondere dentalen, Instruments (2) mit einem Reinigungs- oder Pflegegerät (1, 1', 1") nach einem der vorhergehenden Ansprüche, **dadurch gekennzeichnet, dass**
nach dem Einbringen des Instruments (2) in die Reinigungskammer (3, 3', 3") das Volumen der Reinigungskammer (3, 3', 3") verändert wird.

14. Verfahren zur Reinigung oder Pflege eines medizinischen, insbesondere dentalen, Instruments (2) nach Anspruch 13, **dadurch gekennzeichnet, dass**
zum Verändem des Volumens der Reinigungskammer (3, 3', 3") ein Arbeitsmedium, insbesondere ein Fluid, in Richtung oder an die Außenseite (8, 8'A, 8'B) der flexiblen Außenhülle (5, 5'A, 5'B, 21) gefördert wird.

15. Verfahren zur Reinigung oder Pflege eines medizinischen, insbesondere dentalen, Instruments (2) nach Anspruch 13 oder 14, **dadurch gekennzeichnet, dass**
das Volumen der Reinigungskammer (3, 3', 3") derart verändert wird, dass sich die Form der Reinigungskammer (3, 3', 3") im Wesentlichen der Form des Instruments (2) angleicht oder ihr entspricht oder die flexible Außenhülle (5, 5'A, 5'B, 21) zumindest teilweise am Instrument (2) anliegt.

## Claims

1. A cleaning or care device (1, 1', 1") for cleaning or care of at least one medical, in particular dental, instrument (2) having a cleaning chamber (3, 3', 3") for receiving the at least one instrument (2) to be cleaned and having a media supply (4, 4', 16') through which a cleaning agent or care agent can be conveyed into the cleaning chamber (3, 3', 3"), **characterized in that**
the cleaning chamber (3, 3', 3") is bordered by a flexible outer shell (5, 5'A, 5'B, 21), so that the spatial volume of the cleaning chamber (3, 3', 3") is variable, wherein the flexible outer shell (5, 5'A, 5'B, 21) is attached to a rigid wall (6, 6'), preferably a metallic wall or a wall made of plastic.

2. The cleaning or care device (1, 1', 1") according to Claim 1, **characterized in that**
the rigid wall (6, 6') is designed as a chamber or a housing, which surrounds the flexible outer shell (5, 5'A, 5'B, 21), in particular designed as a chamber or a housing of a cleaning or care unit.

3. The cleaning or care device (1, 1', 1 ") according to Claim 1 or 2, **characterized in that** means are provided for changing the spatial volume of the cleaning chamber (3, 3', 3"), wherein these means for example comprise a conveyance device (7, 7') for conveying a working medium, in particular to the outside (8, 8'A, 8'B) of the flexible outer shell (5'A, 5'B, 21), and/or at least one movable mechanical element.

4. The cleaning or care device (1, 1', 1") according to Claim 3, **characterized in that**
the means for changing the spatial volume of the cleaning chamber (3, 3', 3") comprise a conveyance device (7, 7') for conveying a working medium between the rigid wall (6, 6') and the flexible outer shell (5, 5'A, 5'B, 21), so that the working medium can be conveyed between the rigid wall (6, 6') and the flexible outer shell (5, 5'A, 5'B, 21).

5. The cleaning or care device (1, 1', 1") according to any one of the preceding claims, **characterized in that**
the flexible outer shell (5, 5'A, 5'B, 21) is formed by an elastic film or by an elastic membrane, in particular made of plastic or rubber.

6. The cleaning or care device (1, 1', 1") according to Claim 5, **characterized in that**
the film or membrane is impermeable for water and/or water vapor and/or compressed air and/or microorganisms.

7. The cleaning or care device (1, 1', 1") according to any one of the preceding claims, **characterized in that**
a spacer device is provided for at least partially maintaining a space between the flexible outer shell (5, 5'A, 5'B, 21) and the instrument (2), wherein the spacer device comprises, for example, spacers (10), in particular nubs (10A) or strips provided on the inside (9'A, 9'B) of the flexible outer shell (5, 5'A, 5'B, 21) or a conveyance device (16') for a second working medium opening into the cleaning chamber (3, 3').

8. The cleaning or care device (1, 1', 1") according to any one of the preceding claims, **characterized in that**
means (11, 11') are provided for moving the instrument (2) accommodated in the cleaning chamber (3, 3', 3") relative to the flexible outer shell (5, 5'A, 5'B, 21) to separate surface sections of the instrument (2) from the flexible outer shell (5, 5'A, 5'B, 21) or from a spacer (10).

9. The cleaning or care device (1, 1', 1") according to any one of the preceding claims, **characterized by**
a control and/or regulating device for controlling and/or regulating the conveyance of at least one working medium or for removing at least one working medium.

10. The cleaning or care device (1, 1', 1") according to Claim 9, **characterized in that**
the control and/or regulating device comprises at least one valve and/or at least one sensor, in particular a pressure sensor or a flow sensor, and/or at least one microcontroller.

11. The cleaning or care device (1, 1', 1") according to any one of the preceding claims, **characterized by**
the cleaning or care device (1, 1', 1 ") is designed as a sterilizer, in particular as a steam sterilizer, as a thermal disinfector or as a chemical disinfector.

12. The cleaning or care device (1, 1', 1") according to any one of the preceding claims, **characterized in that**
the cleaning chamber (3, 3', 3") is formed by the interior space of a container (20) that can be introduced into the cleaning or care device (1, 1', 1") and removed from it.

13. A method for cleaning or caring for a medical, in particular dental, instrument (2), having a cleaning or care device (1, 1', 1") according to any one of the preceding claims, **characterized in that**
the volume of the cleaning chamber (3, 3', 3") is changed after introducing the instrument (2) into the cleaning chamber (3, 3', 3").

14. The method for cleaning or care of a medical, in particular dental, instrument (2) according to Claim 13, **characterized in that**
for changing the volume of the cleaning chamber (3, 3', 3"), a working medium, in particular a fluid, is conveyed in the direction of or on the outside (8, 8'A, 8'B) of the flexible outer shell (5, 5'A, 5'B, 21).

15. The method for cleaning or care of a medical, in particular dental, instrument (2) according to Claim 13 or 14, **characterized in that**
the volume of the cleaning chamber (3, 3', 3") is changed such, that the shape of the cleaning chamber (3, 3', 3") essentially adapts to the shape of the instrument (2) or conforms to it or the flexible outer shell (5, 5'A, 5'B, 21) is at least partially in contact with the instrument (2).

## Revendications

1. Appareil de nettoyage ou d'entretien (1, 1', 1") pour le nettoyage ou l'entretien d'au moins un instrument (2) médical, en particulier dentaire, avec une chambre de nettoyage (3, 3', 3") pour réceptionner cet au moins un instrument (2) à nettoyer et avec une alimentation de milieu (4, 4', 16') grâce à laquelle un produit de nettoyage ou d'entretien peut être acheminé dans la chambre de nettoyage (3, 3', 3"), **caractérisé en ce que**
la chambre de nettoyage (3, 3', 3") est délimitée par une enveloppe extérieure flexible (5, 5'A, 5'B, 21) de sorte que le volume spatial de la chambre de nettoyage (3, 3', 3") peut varier, l'enveloppe extérieure flexible (5, 5'A, 5'B, 21) étant fixée sur une paroi (6, 6') rigide, de préférence métallique ou se composant de plastique.

2. Appareil de nettoyage ou d'entretien (1, 1', 1") selon la revendication 1, **caractérisé en ce que**
la paroi (6, 6') rigide est réalisée en tant que chambre ou boîtier, laquelle/lequel entoure l'enveloppe extérieure flexible (5, 5'A, 5'B, 21), en particulier en tant que chambre ou boîtier d'une unité de nettoyage ou d'entretien.

3. Appareil de nettoyage ou d'entretien (1, 1', 1") selon la revendication 1 ou 2, **caractérisé en ce que**
l'on prévoit des moyens pour la modification du volume spatial de la chambre de nettoyage (3, 3', 3"), ces moyens comprenant par exemple un dispositif d'acheminement (7, 7') pour acheminer un milieu de travail, en particulier vers le côté extérieur (8, 8'A, 8'B) de l'enveloppe extérieure flexible (5, 5'A, 5'B, 21) et/ou au moins un élément mécanique mobile.

4. Appareil de nettoyage ou d'entretien (1, 1', 1") selon la revendication 3, **caractérisé en ce que**
les moyens pour la modification du volume spatial de la chambre de nettoyage (3, 3', 3") comprennent un dispositif d'acheminement (7, 7') pour acheminer un milieu de travail entre la paroi rigide (6, 6') et l'enveloppe extérieure flexible (5, 5'A, 5'B, 21), de sorte que le milieu de travail peut être acheminé entre la paroi rigide (6, 6') et l'enveloppe extérieure flexible (5, 5'A, 5'B, 21).

5. Appareil de nettoyage ou d'entretien (1, 1', 1") selon l'une des revendications précédentes, **caractérisé en ce que**
l'enveloppe extérieure flexible (5, 5'A, 5'B, 21) est constituée d'une feuille élastique ou d'une membrane élastique, en particulier en plastique ou en caoutchouc.

6. Appareil de nettoyage ou d'entretien (1, 1', 1") selon la revendication 5, **caractérisé en ce que**
la feuille ou la membrane est imperméable à l'eau et/ou à la vapeur d'eau et/ou à l'air comprimé et/ou aux micro-organismes.

7. Appareil de nettoyage ou d'entretien (1, 1', 1") selon l'une des revendications précédentes, **caractérisé en ce que**
l'on prévoit un dispositif d'écartement pour l'espacement au moins partiel de l'enveloppe extérieure flexible (5, 5'A, 5'B, 21) par rapport à l'instrument (2), le dispositif d'écartement comprenant par exemple des écarteurs (10) prévus sur le côté intérieur (9'A, 9'B) de l'enveloppe extérieure flexible (5, 5'A, 5'B, 21), en particulier des grosseurs (10A) ou bordures, ou bien un dispositif d'acheminement (16') débouchant dans la chambre de nettoyage (3, 3') pour un deuxième milieu de travail.

8. Appareil de nettoyage ou d'entretien (1, 1', 1") selon l'une des revendications précédentes, **caractérisé en ce que**
l'on prévoit des moyens (11, 11') pour faire bouger l'instrument (2) réceptionné dans la chambre de nettoyage (3, 3', 3") par rapport à l'enveloppe extérieure flexible (5, 5'A, 5'B, 21) afin de séparer des parties de surface de l'instrument (2) de l'enveloppe extérieure flexible (5, 5'A, 5'B, 21) ou d'un écarteur (10).

9. Appareil de nettoyage ou d'entretien (1, 1', 1") selon l'une des revendications précédentes, **caractérisé par**
un dispositif de commande et/ou de réglage pour la commande et/ou le réglage de l'acheminement d'au moins un milieu de travail ou pour évacuer au moins un milieu de travail.

10. Appareil de nettoyage ou d'entretien (1, 1', 1") selon la revendication 9, **caractérisé en ce que**
le dispositif de commande et/ou de réglage comprend au moins une soupape et/ou au moins un capteur, en particulier un capteur de pression ou un capteur de débit, et/ou au moins un microcontrôleur.

11. Appareil de nettoyage ou d'entretien (1, 1', 1") selon l'une des revendications précédentes, **caractérisé en ce que**
l'appareil de nettoyage ou d'entretien (1, 1', 1") est réalisé en tant que stérilisateur, en particulier en tant que stérilisateur à vapeur, en tant que thermodésinfecteur ou en tant que désinfecteur chimique.

12. Appareil de nettoyage ou d'entretien (1, 1', 1") selon l'une des revendications précédentes, **caractérisé en ce que**
la chambre de nettoyage (3, 3', 3") est formée par l'espace intérieur d'un conteneur (20) pouvant être introduit dans l'appareil de nettoyage ou d'entretien (1, 1', 1") et pouvant en être ressorti.

13. Procédé pour le nettoyage ou l'entretien d'un instrument (2) médical, en particulier dentaire, avec un appareil de nettoyage ou d'entretien (1, 1', 1") selon l'une des revendications précédentes, **caractérisé en ce que**,
après avoir introduit l'instrument (2) dans la chambre de nettoyage (3, 3', 3"), le volume de la chambre de nettoyage (3, 3', 3") est modifié.

14. Procédé pour le nettoyage ou l'entretien d'un instrument (2) médical, en particulier dentaire, selon la revendication 13, **caractérisé en ce que**,
pour modifier le volume de la chambre de nettoyage (3, 3', 3"), un milieu de travail, en particulier un fluide, est acheminé en direction de l'enveloppe extérieure flexible (5, 5'A, 5'B, 21) ou vers le côté extérieur (8, 8'A, 8'B) de celle-ci.

15. Procédé pour le nettoyage ou l'entretien d'un instrument (2) médical, en particulier dentaire, selon la revendication 13 ou 14, **caractérisé en ce que**
le volume de la chambre de nettoyage (3, 3', 3") est modifié de telle sorte que la forme de la chambre de nettoyage (3, 3', 3") ressemble sensiblement à la forme de l'instrument (2) ou lui correspond ou que l'enveloppe extérieure flexible (5, 5'A, 5'B, 21) repose au moins partiellement contre l'instrument (2).
